# EUROPEAN PATENT APPLICATION

(11) **EP 2 705 949 A1**
(43) Date of publication of application: **12.03.2014**
(21) Application number: 12183608.4
(22) Date of filing: 07.09.2012
(51) Int. Cl.: B31F 1/36, B31F 1/28, B31D 5/00, A61M 16/10, A62B 9/00

(54) **Heat and moisture exchange media**

(71) Applicant: Intersurgical UAB, 18170 Pabrade (LT)
(72) Inventor: Davies Martin, Wokingham, Berkshire RG41 2RZ (GB)
(74) Representative: Adamson Jones

(57) **Abstract**

Methods of embossing a HME material, manufacturing a HME medium, and manufacturing a HME device, are disclosed, which each comprise the steps of exposing a HME material to a temperature of at least 40ºC, and urging the HME material against a patterned surface. An embossed HME material having a tensile strength of less than 9.0 N/mm is also disclosed.

## Description

This invention relates to methods of manufacturing heat and moisture exchange (HME) media, and particularly HME media for inclusion into breathing lines.

During normal breathing, gas enters the body through the mouth and/or nose and is heated and humidified as it passes through the airways and into the lungs. However, in cases such as artificial ventilation with a respirator or anaesthesia apparatus, the heating and humidification of inhaled gas before it reaches the lower airways and lungs may be inadequate. The exposure of the lower airways and lungs to this inadequately heated and humidified gas may have undesirable effects, such as impairment of mucosal function and drying or irritation of mucosal surfaces, potentially leading to discomfort and increased susceptibility to respiratory tract infection.

One means of overcoming the problem is by passive heating and humidification of gas prior to inhalation with the use of a HME device. Such devices generally comprise a housing having two ports located at opposite ends of a chamber, with the chamber containing an HME medium formed of HME material that is capable of retaining and releasing heat and moisture. This HME material is typically a layer of cellulose paper that includes a hygroscopic additive to enhance the ability of the HME material to retain moisture.

HME media are typically formed of a long strip of HME material wound into a coil, which is positioned in the chamber of the HME device such that the direction of gas flow between the two ports is along the longitudinal axis of the coil, with the gas thereby passing through the gaps between adjacent layers of the HME material.

The HME device may be included into a breathing line such that exhaled gas and gas for inhalation passes through the HME medium. When gas that is exhaled by the patient passes through the HME medium, heat and moisture picked up by the gas in the airways of the patient is transferred to and retained by the HME medium. As gas for inhalation subsequently passes through the HME medium in the opposite direction, heat and moisture is transferred from the HME medium to the gas before it enters the airways of the patient.

The ability of the HME medium to effectively transfer heat and moisture from exhaled gas to gas for inhalation in this way depends on a number of factors, including the surface area of the HME medium that is available for heat and moisture exchange, and the content of hygroscopic material in the HME medium. In particular, HME materials having a higher hygroscopic material content generally have better heat and moisture exchange performance.

HME material is generally embossed with a pattern, most commonly a pattern of corrugations, prior to being formed into a HME medium. In particular, the embossing of the HME material increases the surface area that is available for heat and moisture exchange, thereby improving heat and moisture exchange performance of the HME medium. Furthermore, in cases where the HME medium is in the form of a coil of HME material, the embossed pattern serves to increase the separation between adjacent layers of the HME material in the coil, thereby improving gas flow through the HME medium and hence reducing resistance to gas flow.

The conventional process for embossing HME material involves applying pressure to the HME material between two meshing patterned surfaces, in particular by passing the HME material through the nip of a pair of rollers having a meshing pattern. However, HME material is prone to tearing or breakage during this embossing process, in particular as a result of the relatively high pressure that must be applied to the HME material in order to generate an embossed pattern of sufficient depth. This tearing or breakage is a particular problem in relation to HME materials having a high hygroscopic material content, as such materials tend to have lower tensile strength and tear-resistance. However, some hygroscopic materials have a greater effect on the tensile strength and tear-resistance of HME materials that others.

Accordingly, the relatively high pressure that is required to generate an embossed pattern of sufficient depth results in tearing and breakage of the HME material during the embossing process, and in particular has limited the content and type of hygroscopic material that may be included into HME material, and hence the heat and moisture exchange performance of HME media.

Accordingly, there has now been devised an improved method of manufacturing HME media that overcomes or substantially mitigates the above-mentioned and/or other problems associated with the prior art.

Thus, according to a first aspect of this invention, there is provided a method of embossing a HME material comprising the steps of exposing the HME material to a temperature of at least 40ºC, and urging the HME material against a patterned surface.

Exposing the HME material to a temperature of at least 40ºC enables an embossed pattern of sufficient depth to be generated by urging the HME material against the patterned surface at a lower pressure. Accordingly, the method according to the first aspect of this invention enables HME material to be effectively embossed with a lower incidence of breakage or tearing. In particular, it is believe that exposing the HME material to a temperature of at least 40ºC plasticises the fibres of the HME material and/or reduces the force required for the fibres to move relative to one another in the HME material.

The HME material may be exposed to a temperature of at least 40ºC before being urged against the patterned surface. In this case, the HME material is preferably exposed to a temperature of at least 40ºC immediately before being urged against the patterned surface. The HME material may be exposed to gas at a temperature of least 40ºC. This may be achieved by passing the HME material through a chamber containing gas at a temperature of least 40ºC, such as a chamber comprising heating elements such as electrical heating elements. The HME material may also be exposed to a stream of gas at a temperature of at least 40ºC. Alternatively, the HME material may be exposed to infra red radiation, or may be contacted by a surface at a temperature of at least 40ºC.

However, the HME material is preferably exposed to a temperature of at least 40ºC and urged against the patterned surface simultaneously. In particular, this may be achieved by the patterned surface being at a temperature of at least 40ºC.

The HME material manufactured according to this invention may be for the manufacture of HME media for inclusion into a breathing line, such as a breathing circuit for artificial ventilation by a respirator or anaesthesia apparatus.

The pressure at which the HME material is urged against the patterned surface is sufficient to generate an embossed pattern of sufficient depth. The pressure required to generate an embossed pattern of sufficient depth may vary significantly depending on the properties of the HME material, and particularly its thickness and resilience. However, the temperature of the patterned surface being at least 40ºC enables an embossed pattern of sufficient depth to be generated using a lower pressure than would otherwise be the case. In particular, the pressure required to generate an embossed pattern of sufficient depth is preferably reduced by at least 5%, at least 10%, at least 20%, at least 30%, at least 40%, at least 50%, at least 60% or at least 70%.

The pressure at which the HME material is urged against the patterned surface may be less than 2.0 bar, less than 1.8 bar, less than 1.5 bar or less than 1.3 bar. Furthermore, the pressure at which the HME material is urged against the patterned surface may be more than 0.3 bar, more than 0.5 bar or more than 0.8 bar. In particular, the pressure at which the HME material is urged against the patterned surface is typically around 1.0 bar.

It is believed that the reduced tensile strength and tear-resistance of HME materials having a high hygroscopic material content is at least partially due to the relatively high equilibrium moisture levels of such materials. In particular, it is believed that this increased level of moisture disrupts interactions, such as hydrogen bonding, between the fibres of the HME material. Accordingly, the method of this invention may include a step of reducing the moisture content of the HME material prior to embossing. In particular, the HME material may be exposed to an environment of low humidity, or heated, for example by passing the HME material over a heated surface, such as the surface of a roller, or by exposing it to infra red radiation. In any case, the method of this invention preferably avoids introducing moisture into the HME material prior to embossing.

The embossed pattern that is generated on the HME material is dependent on the pattern of the patterned surface. The method of this invention enables the HME material to be embossed with any desired pattern, although the embossed pattern is preferably a pattern of corrugations. Such corrugations are preferably straight and may be aligned either perpendicular or at an oblique angle to the axis of the HME material. Alternatively, the corrugations may be "V" shaped, in a zigzag arrangement or curved.

The HME material may be urged against the patterned surface by any suitable means. However, the HME material is preferably urged against the patterned surface by another surface. In particular, the HME material may be placed under pressure between two surfaces, one of those surfaces being the patterned surface. In this case, the HME material may be exposed to a temperature of at least 40ºC by either or preferably both of the surfaces being heated to at least 40ºC.

However, in a preferred ambodiment, both of the surfaces may be patterned, in which case the HME material is placed under pressure between a first patterned surface and a second patterned surface. In particular, the first and second patterned surfaces may have corresponding patterns that are able to mesh with one another. In this case, either or preferably both patterned surfaces may be heated to at least 40ºC.

The temperature of the patterned surface or surfaces is preferably less than the temperature that would cause thermal decomposition of the HME material upon contact. The temperature of the patterned surface or surfaces is preferably less the 100ºC, more preferably less than 90ºC, yet more preferably less than 80ºC and most preferably less than 70ºC. The temperature of the patterned surface or surfaces is preferably greater than 40ºC, more preferably at least 50ºC and most preferably 60ºC. In particular, the temperature of the patterned surface or surfaces is preferably between 60ºC and 70ºC and is most preferably around 65ºC.

In a preferred embodiment of this invention, the two surfaces are preferably the circumferential surfaces of a first roller and a second roller. In particular, the first patterned surface and the second patterned surface are formed by at least part of the circumferential surfaces of the first roller and a second roller respectively. In particular, the HME material may be passed through a nip between the patterned surfaces of the first and second rollers, with the HME material being placed under sufficient pressure at the nip to generate an embossed pattern. This arrangement is particularly preferred as it facilitates the performance of the method of this invention as a continuous process by continuously passing HME material through the nip.

The circumferential surfaces of first and second rollers preferably comprise patterned surfaces in the form a series of adjacent ridges (ie geared surfaces), which may form a pattern of corrugations in the HME material. In particular, the rollers may comprise normal or spur geared surfaces in which the ridges are straight and aligned parallel to the axis of rotation of the rollers, such that the corrugations are perpendicular to the axis of the HME material. The rollers may comprise helical geared surfaces, in which the ridges are aligned at an oblique angle to the axis of rotation of the rollers, such that the corrugations are at an oblique angle to the axis of the HME material. The rollers may comprise geared surfaces that are capable of forming "V" shaped, zigzag or curved corrugations.

The patterned surface is preferably formed of a material that is sufficiently hard to enable effective embossing of the HME material, and is sufficiently conductive to enable effective and even heating of the patterned surface. Particularly suitable materials for forming the patterned surface are metals. A preferred metal is steel, such as stainless steel and in particular grade AISI 420 steel.

The temperature of the patterned surface may be adjusted by any suitable means. In particular, a heating means is generally required to bring the temperature of the patterned surface to the desired temperature, and in particular at least 40ºC.

The heating means may take any suitable form. The heating means may be integral to the patterned surface, or may be separate from the patterned surface. The heating means may generate a region of heated gas, preferably having low moisture content, which may be arranged to heat the HME material before the material is urged against the patterned surface. The region of heated gas may be a stream of gas that is directed to transfer heat to the HME material, for example by heating the patterned surface. The stream of heated gas is preferably not directed to a region of the patterned surface against which the HME material is urged so as not to interfere with the embossing process. Where the patterned surface is formed of an electrically conductive material, such as steel, the heating means may comprise an induction heater arranged to heat the patterned surface by induction heating. The heating means may comprise one or more heating elements, such as electrical heating elements and in particular cartridge heaters, positioned to transfer heat to the patterned surface. By "heated" is meant heated relative to ambient temperatures.

A temperature monitoring means, such as a thermocouple or a non-contact temperature sensor, such as an infra-red thermometer, may monitor the temperature of the patterned surface. The temperature monitoring means may cooperate with the heating means by way of a feedback system, such as a PID controller, in order to regulate the temperature of the patterned surface.

The material on which the patterned surface is formed may further comprise a heat exchange surface. The heat exchange surface may be adapted to improve the efficiency of heat exchange with the heating means, such as by increasing the surface area available for heat exchange and/or reducing the volume of material and hence the amount of heat energy required to bring the patterned surface to the desired temperature.

When the patterned surface is the circumferential surface of a roller, the heat exchange surfaces may be formed on the end faces of the roller. These heat exchange surfaces may comprise a recess, and in particular an annular groove circumscribing the centre of the end face. The heating means may be arranged to transfer heat to the surface of the annular groove. In particular, the heating means may be accommodated within the annular groove. In this case, the heating means is preferably a heating element positioned in close proximity to the surface of the annular groove. The heating means is preferably not placed in direct contact with the roller so as to avoid obstructing the rotation of the roller.

The HME material is generally in the form of a sheet with a thickness that is great enough to retain sufficient heat and moisture, while still providing sufficient surface area for heat and moisture exchange. In particular, the HME material preferably has a thickness of between 0.01 mm and 1.0mm, more preferably between 0.05mm and 0.5mm, and most preferably between 0.1 mm and 0.3mm and in particular about 0.2mm.

The HME material may be formed of any material that is capable of retaining and releasing heat and moisture, but is generally formed of a fibrous material, such as paper and particularly cellulose paper. However, the HME material may be formed of, or also include, other plant fibres or synthetic fibres such as polyamine, polyester, polyurethane, polyacrylonitrile and polyvinyl alcohol, or any combination of the above.

The HME material may include additives, such as hygroscopic materials that improve the ability of the HME material to retain moisture. In particular, suitable hygroscopic materials include polyols such as glycols and glycerine, hygroscopic polymers such as polyvinylpyrrolidone, polyacrylic acid and polyvinyl alcohol, and hygroscopic salts such as polyacrylate, calcium chloride, potassium chloride and lithium chloride, or any combination of the above.

The embossed pattern generally has a depth that is sufficient to increase the surface area of the HME material that is available for heat and moisture exchange. In particular, the embossed pattern preferably has a depth of between 0.1 mm and 3.0mm, more preferably between 0.2mm and 2.0mm, and most preferably between 0.5mm and 1.0mm.

The method of embossing HME material according to first aspect of this invention enables HME material having a lower tensile strength to be effectively embossed.

Thus, according to a second aspect of this invention, there is provided a embossed HME material having tensile strength of less than 9.0 N/mm.

The tensile strength of the embossed HME material may less than 8.0 N/mm, less than 7.0 N/mm, less than 6.0 N/mm or less than 5.0 N/mm.

HME material having greater hygroscopic material content has enhanced ability to retain and release moisture, and hence has improved heat and moisture exchange performance. The hygroscopic material content of the HME material may be greater than 50%, at least 55%, at least 60%, at least 65%, at least 70%, at least 75% or at least 80% by weight.

In cases where the HME material is in the form of a sheet, the hygroscopic material content may be at least 100g/m², at least 120g/m², at least 140g/m², at least 160g/m², at least 180g/m² or at least 200g/m².

The HME material is preferably in the form of elongate strips, which facilitate the performance of the embossing process, and furthermore facilitates the conversion of the embossed HME material into HME media.

Thus, according to a third aspect of this invention, there is provided a method of manufacturing a HME medium comprising the steps of
providing a HME material;
embossing the HME material according to the first aspect of this invention; and
forming the embossed HME material into a HME medium.

The HME medium manufactured according to this invention may be for use in the manufacture of HME media for inclusion into a breathing line, such as a breathing circuit for artificial ventilation by a respirator or anaesthesia apparatus.

The HME material is preferably provided in the form of strips, preferably have a width of between 5mm and 20mm, and in particular around 15mm. However, the HME material may be formed into strips following the embossing step. Strips of HME material are generally suitable for forming into HME media.

The method of embossing HME material according to first aspect of this invention enables HME material having a higher content of hygroscopic material to be effectively embossed, thereby enabling the manufacture of HME media having improved heat and moisture exchange performance.

Thus, according to a fourth aspect of this invention, there is provided a HME medium comprising an embossed HME material according to the second aspect of this invention.

HME media are preferably substantially free from microbial contamination. This may be achieved by carrying out the method in a sterile or aseptic environment. Alternatively, the method may comprise an additional step sterilising the HME material, for example by gamma irradiation or exposure of the HME material to ethylene oxide.

The embossed HME material may be formed into a HME medium by winding the HME material into a coil, in which case the embossed pattern serves to increase the separation between adjacent layers of the HME material in the coil, thereby improving gas flow through the HME medium and hence reducing resistance to gas flow. This may be carried out by attaching a strip of embossed HME material to a spindle and rotating the spindle such that the embossed HME material is wound into a coil around the spindle. The resulting HME medium may then be removed from the spindle and the process repeated.

Each HME medium may be formed of multiple layers of HME material formed into a coil, such as five, four, three and preferably two layers of HME material adjacent to one another. In this case, the corrugations of the adjacent layers of HME material are unable to mesh, thereby maintaining the separation of adjacent layers of the coil and hence improving gas flow through the HME media. This may be achieved by the adjacent layers of the HME material being embossed with different embossed patterns. In a preferred embodiment, the HME medium is formed of a coil of two adjacent layers of HME material, in which the corrugations of each layer of HME material are offset from the axis of the HME material in opposite orientations.

The height of the HME media corresponds to the width of the strip or strips of embossed HME material used to form it, and hence is generally between 5mm and 20mm, and in particular around 15mm.

The diameter of the HME media depends on the thickness of the HME material, the number of revolutions in the coil and the separation between the layers of the coil. In particular, the diameter of the HME media may be from 1.5cm to 8.0cm, more preferably around 3.0cm to 7.0cm, most preferably 5.0cm to 6.0cm, and in particular around 5.5cm.

HME media manufactured according to this invention are particularly suitable for the manufacture of HME devices for use in a breathing line, such as a breathing circuit for artificial ventilation by a respirator or anaesthesia apparatus.

Thus, according to a fifth aspect of this invention, there is provided a method of manufacturing a HME device comprising the steps of
manufacturing a HME medium according to the third aspect of this invention;
providing a housing having a chamber and two ports in communication with the chamber; and
introducing the HME medium into the chamber.

The HME device may be included in a breathing line. In particular, one port may be attached to a source of gas, such as a respirator or anaesthesia apparatus, or be open to ambient air. Another port may be attached to a part for engaging the airways of a patient, such as a breathing mask, endotracheal tube or tracheotomy tube.

Accordingly, gas that is exhaled by the patient passes through the HME medium, and heat and moisture that has been picked up by the gas in the airways of the patient is transferred to and retained by the HME medium. As gas for inhalation then passes through the HME medium in the opposite direction, heat and moisture is transferred from the HME medium to the gas before it enters the airways of the patient. The method of this invention enables the manufacture of HME media having a higher hygroscopic material content, and hence enables greater heat and moisture exchange performance to be achieved.

Thus, according to a sixth aspect of this invention, there is provided a HME device comprising a HME medium according to the fourth aspect of this invention.

The housing may be formed of any suitable material, but is preferably formed of injection moulded plastic. The ports are preferably located at opposite ends of the chamber and the chamber preferably has a greater transverse cross-sectional area than the ports.

The HME device is preferably arranged such that substantially all gas flow between the two ports passes through the HME medium in the chamber. Where the HME medium is in the form of a coil, the HME medium is preferably positioned in the chamber such that gas flow between the two ports is along the longitudinal axis of the coil, with the gas passing through the gaps between adjacent layers of the HME material.

The chamber preferably comprises a space adjacent to each port that is not occupied by the HME medium, which allows expansion of the gas upon entry into the chamber. This feature enables gas flow to be distributed substantially evenly throughout the HME medium.

Some or all of the material of the housing may be transparent, or substantially transparent, to enable visual inspection of the HME medium.

The housing may comprise additional ports. For example, the housing may comprise a sampling port for sampling gas in the chamber, which preferably includes a valve or closure for preventing gas flow through the sampling port during normal operation of the HME device.

Each port preferably comprises standard connectors, such as Luer tapers, that are compatible with conventional breathing lines and associated apparatus.

According to a seventh aspect of this invention, there is provided an apparatus for embossing HME material comprising a first roller, at least part of the circumferential surface of which comprises a first patterned surface, and a second roller, at least part of the circumferential surface of which comprises a second patterned surface, the first roller and the second roller defining a nip at which the first patterned surface and the second patterned surface mesh, wherein the apparatus further comprises a means for exposing HME material to a temperature of at least 40ºC.

The means for exposing HME material to a temperature of at least 40ºC may comprise a means for bringing the temperature of the first patterned surface and/or second patterned surface to at least 40ºC.

The means for adjusting the temperature of each patterned surface is generally a heating means, which may be of the type described above.

A currently preferred embodiment of the invention will now be described, by way of illustration only, with reference to the accompanying drawings, in which:
Figure 1 is a schematic representation, not to scale, of an apparatus for manufacturing HME media; and,
Figure 2 is a perspective view of a corrugation roller for use in the apparatus depicted in Figure 1.

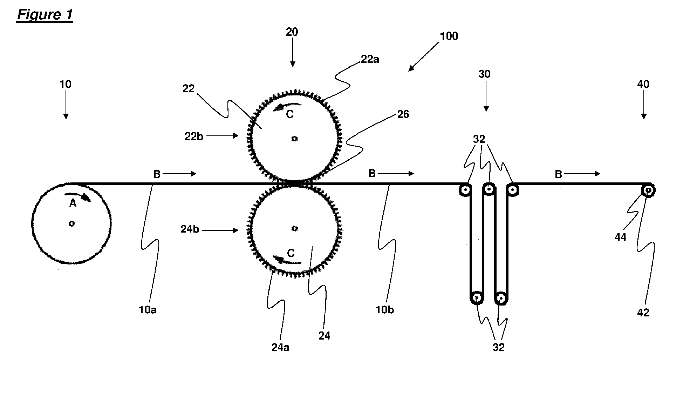

Referring first to Figure 1, an apparatus for manufacturing HME media is generally designated 100. The apparatus 100 comprises a source reel 10 carrying a strip of HME material 10a, a corrugation assembly 20, a tension control assembly 30, and a winding assembly 40.

The strip of HME material 10a has a width of approximately 15mm and a thickness of approximately 0.2mm. The strip of HME material 10a is unwound by the rotation of the source reel 10 in the direction of arrow A and is transferred along the apparatus 100 in the direction of arrow B towards the corrugation assembly 20.

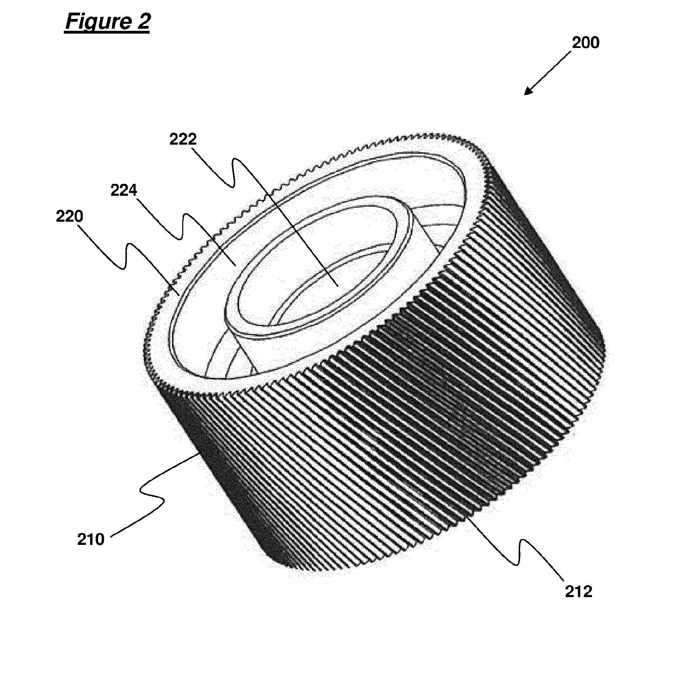

The corrugation assembly 20 comprises an upper corrugation roller 22 and a lower corrugation roller 24 that together define a nip 26. A motor (not shown) may drive the rotation of the corrugation rollers 22,24 in the direction or arrows C. The circumferential surfaces 22a,24a of the corrugation rollers 22,24 comprise a series of adjacent ridges that are offset from the axis of rotation of the corrugation rollers 22,24 by 20º (see Figure 2). The ridges of the upper corrugation roller 22 mesh with the ridges of the lower corrugation roller 24 at the nip 26.

Heating means 22b,24b are able to apply heat to the corrugation rollers 22,24. The heating means 22b,24b comprises cartridge heaters that are positioned to apply heat to the corrugation rollers 22,24. The heating means also include a thermocouple that forms a feedback loop with the cartridge heaters by way of a PID controller in order to regulate the temperature of the patterned surface.

In use the heating means 22b,24b apply heat to the corrugation rollers 22,24 such that their circumferential surfaces 22a,24a are heated to between 60ºC and 70ºC. The strip of HME material 10a reaches the corrugation assembly 20 and is drawn into the nip 26 by the rotation of the corrugation rollers 22,24 in the direction of arrows C. As the strip of HME material 10a passes through the nip 26 it is compressed between the meshing ridges on the circumferential surfaces 22a,24a of the corrugation rollers 22,24 and is thereby formed into a corrugated HME material 10b. The corrugations of the corrugated HME material 10b correspond to the ridges on the circumferential surfaces 22a,24a of the corrugation rollers 22,24 and hence are offset from the transverse axis of the strip of corrugated HME material 10b by 20º.

The pressure applied to the strip of HME material 10a at the nip 26 is around 0.5 bar. This pressure is able to generate corrugations on the strip of HME material 10a of sufficient depth because the temperature of the circumferential surfaces 22a,24a of the corrugation rollers 22,24 is between 60ºC and 70ºC.

The corrugated strip of HME material 10b then passes out of the nip 26 and enters the tension control assembly 30, which comprises a number of rollers 32 that control the tension of the HME material 10a, 10b as it passes through the nip 26.

It should be appreciated that the corrugated strip of HME material 10b may be wound directly onto another roller after passage through the tension control assembly 30, and processed into HME media in a separate process. However, in this case, the corrugated strip of HME material 10b is immediately processed into HME media by the winding assembly 40.

The winding assembly comprises a spindle 42 to which the corrugated strip of HME material 10b is attached. The spindle 42 may then be rotated in order to wind the corrugated strip of HME material 10b into a coil 44 as it passes out of the tension control assembly 30. When the coil 44 reaches the desired diameter, the corrugated strip of HME material 10b is cut and the coil 44 is removed from the spindle 42. The resulting free end of the corrugated strip of HME material 10b may then be attached to the spindle 42 and the process repeated. The coil 44 may be introduced into a HME device housing.

Typically this process will be carried out in tandem with a second apparatus (not shown) running in parallel to the first 100, resulting in a second corrugated strip of HME material (not shown) being generated in addition to the first 10b. In this case, both corrugated strips of HME material would be attached to the spindle 42 and wound into a coil 44 as described above. However, the corrugations of the second corrugated strip of HME material are offset from the transverse axis of the strip by 20º in the opposite orientation to the corrugations of the first strip of corrugated HME material 10b. This arrangement prevents the corrugations of the corrugated strips of HME material from meshing and hence maintains the separation of adjacent layers of the coil 44, thereby reducing the resistance of the coil 44 to gas flow after is has been introduced into a HME device housing.

Referring now to Figure 2, a corrugation roller is generally designated 200. The corrugation roller 200 is generally the shape of a short cylinder comprising a circumferential surface 210 and two generally circular faces 220. The circumferential surface 210 is made up of adjacent parallel ridges 212 having a separation roughly equal to their width. The ridges 212 are offset from the axis of the corrugation roller 200 by 20º, which gives the corrugation roller 200 the general appearance of a helical gear.

The corrugation roller 200 further comprises a passageway 222 passing through the centre of the corrugation roller 200 between the circular faces 220. Each circular face 220 further comprises a deep groove 224 that circumscribes the passageway 222. The groove 224 increases the surface area of the corrugation roller 200 that is available for heat transfer with the heating means, and furthermore reduces the amount of heat energy that is required to bring the corrugation roller 200 to the desired temperature.

The corrugation roller 200 may engage an apparatus for manufacturing HME media 100 as depicted in Figure 1 via an axle passing through the passageway 222. The rotation of the corrugation roller 200 may be driven by a motor acting upon the axle. The corrugation roller 200 may be positioned relative to another roller such that both rollers together define a nip, as depicted in Figure 1. In this arrangement, the ridges 212 of the circumferential surface 210 of the corrugation roller 200 should engage corresponding ridges on the other rollers, such that the ridges of the rollers mesh as they are rotated. In particular, in order to mesh effectively with the ridges 212 of the corrugation roller 200, the ridges of the other roller should have roughly the same dimensions and separation, and be offset from the axis of the that roller by 20º in the opposite orientation.

The temperature of the corrugation roller 200 may be adjusted by a heating means as described above. The heating is accommodated within the groove 224 in in close proximity to the surface of the groove 224 as the roller 200 rotates. In particular, the heating means is not in direct contact with the roller 200 in order to avoid obstruction of the rotation of the roller.

## Claims

1. A method of embossing a HME material comprising the steps of exposing the HME material to a temperature of at least 40ºC, and urging the HME material against a patterned surface.

2. A method according to Claim 1, wherein the HME material is for the manufacture of HME media for inclusion into a breathing line.

3. A method according to Claim 1 or Claim 2, wherein the pressure required to generate an embossed pattern of sufficient depth is reduced by at least 5%, at least 10%, at least 20%, at least 30%, at least 40%, at least 50%, at least 60% or at least 70%.

4. A method according to any preceding claim, wherein the temperature of the patterned surface is less the 100ºC, less than 90ºC, less than 70ºC or less than 60ºC.

5. A method according to any preceding claim, wherein the temperature of the patterned surface is greater than 40ºC, at least 50ºC or at least 60ºC.

6. A method according to any preceding claim, wherein the temperature of the patterned surface is between 60ºC and 70ºC, or around 65ºC.

7. A method according to any preceding claim, wherein the patterned surface is a pattern of corrugations.

8. A method according to any preceding claim, wherein the HME material is heated by a region of heated gas, before the material is urged against the patterned surface.

9. A method of manufacturing a HME medium comprising the steps of:
providing a HME material;
embossing the HME material according to Claim 1; and
forming the embossed HME material into a HME medium.

10. A method of manufacturing a HME device comprising the steps of:
manufacturing a HME medium according to Claim 9;
providing a housing having a chamber and two ports in communication with the chamber; and,
introducing the HME medium into the chamber.

11. An embossed HME material having a tensile strength of less than 9.0 N/mm.

12. An embossed HME material according to Claim 11, wherein the HME material has a tensile strength of less than 8.0 N/mm, less than 7.0 N/mm, less than 6.0 N/mm or less than 5.0 N/mm.

13. An apparatus for embossing HME material comprising a first roller, at least part of the circumferential surface of which comprises a first patterned surface, and a second roller, at least part of the circumferential surface of which comprises a second patterned surface, the first roller and the second roller defining a nip at which the first patterned surface and the second patterned surface mesh, wherein the apparatus further comprises a means for exposing HME material to a temperature of at least 40ºC.

14. An apparatus according to Claim 13, wherein the means for exposing HME material to a temperature of at least 40ºC may comprise a means for bringing the temperature of the first patterned surface and/or second patterned surface to at least 40ºC.

15. An apparatus according to Claim 13 or Claim 14, wherein the means for adjusting the temperature of each patterned surface is a heating means.
